# EUROPEAN PATENT SPECIFICATION

(11) **EP 3 532 165 B1**
(45) Date of publication and mention of the grant of the patent: **24.02.2021**
(21) Application number: 17791056.9
(22) Date of filing: 25.10.2017
(51) Int. Cl.: A61Q 5/00, A61Q 5/12, A61K 8/81

(54) **USE OF CATIONIC POLYMER HAVING QUATERNARY AMMONIUM GROUPS FOR THE PROTECTION OF HAIR AGAINST UV DAMAGE**
VERWENDUNG EINES KATIONISCHEN POLYMERS MIT QUATERNÄREN AMMONIUMGRUPPEN ZUM SCHUTZ VON HAAR GEGEN UV-BESCHÄDIGUNG
UTILISATION DE POLYMÈRE CATIONIQUE PRÉSENTANT DES GROUPES D'AMMONIUM QUATERNAIRE POUR LA PROTECTION DES CHEVEUX CONTRE LES DOMMAGES DUS AUX ULTRAVIOLETS

(30) Priority: 25.10.2016 EP 16195591
(43) Date of publication of application: 04.09.2019
(73) Proprietor: DSM IP Assets B.V., 6411 TE Heerlen (NL)
(72) Inventor: MENDROK-EDINGER, Christine, 4303 Kaiseraugst (CH); HECKER, Karina, 4303 Kaiseraugst (CH)
(74) Representative: Dux, Roland
(86) International application number: PCT/EP2017/077288
(87) International publication number: WO 2018/077939

(56) References cited:
- WO-A1-2004/043412
- CN-A- 103 432 023
- "Final report on the safety assessment of polyquaternium-7", JOURNAL OF THE AMERICAN COLLEGE OF TOXICOLOGY, NEW YORK, NY, US, vol. 14, no. 6, 1 January 1995 (1995-01-01), pages 476-484, XP009192612, ISSN: 0730-0913
- HERRWERTH S ET AL: "Testing polysilicone-19 for hair conditioning and uv protection claims", COSMETICS & TOILETRIES,, vol. 123, no. 5, 1 May 2008 (2008-05-01), pages 101-110, XP009134523, ISSN: 0361-4387
- CRODA INC: "Use of diester quats for UV protection", RESEARCH DISCLOSURE, MASON PUBLICATIONS, HAMPSHIRE, GB, vol. 535, no. 33, 1 November 2008 (2008-11-01), page 982, XP007138562, ISSN: 0374-4353
- Apoorva Mahajan ET AL: "Advancements in polymers used in hair care: a review", International Journal of Research in Cosmetic Science, 29 February 2016 (2016-02-29), XP055323994, Retrieved from the Internet: URL:http://urpjournals.com/tocjnls/32_16v6 i1_2.pdf [retrieved on 2016-11-29]

## Description

### Technical Field

The present invention relates to the UV protection of hair.

### Background of the invention

UV radiation is known to have a detrimental effect not only on skin but also on hair. The UV light leads to a damage of the keratin and the structure of cuticle composition. This results particular in dull and lifeless looking hair.

Such UV damage is traditionally reduced by the use of UV filters, which absorb the detrimental UV irradiation.

However, UV filters, are very expensive and their use is often limited by their solubility, respectively compatibility, in a cosmetic preparation. Therefore, it is a desire to have an efficient UV protection at a competitive lower price level without formulation restrictions.

Cationic polymers have been known and used already for several years in the field of cosmetics. Particularly, the polyquaternium compounds are used as film formers, as conditioning agents and as antistatic agents. Polymers in hair care are reviewed in Apoorva Mahajan ET AL: "Advancements in polymers used in hair care: a review",International Journal of Research in Cosmetic Science, 2016.

### Summary of the invention

The inventors of the present invention have surprisingly found that a very specific class of cationic polymers having quaternary ammonium groups can be used for the protection of hair against UV damage.

Treatment of hair with these homopolymers and copolymers leads to the fact that after UV exposure particularly a higher denaturation temperature of hair as well as, to a lower extent, a higher denaturation enthalpy can be measured by DSC reflecting better hair integrity. It has been found that these homopolymers and copolymers protect the hair particularly against the UVB radiation (280-315 nm).

This effect of UV protection is particularly surprising as this specific class of cationic polymers having quaternary ammonium groups has no suitable chromophores in its chemical structure which might absorb the UV radiation.

Further aspects of the invention are subject of further independent claims. Particularly preferred embodiments are subject of dependent claims.

### Detailed description of the invention

In a first aspect, the present invention relates to a use of a cationic polymer having quaternary ammonium groups which is either a homopolymer of 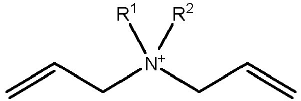 or a copolymer of 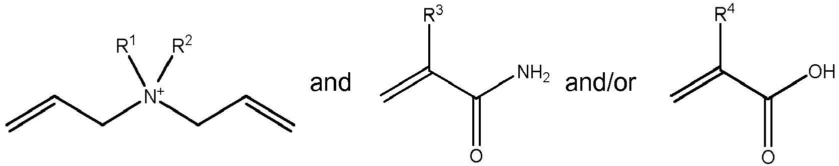 for the protection of hair against UV damage;
wherein
R¹ and R² represent independently from each other either a linear or a branched C₁₋₁₀-alkyl group, particularly a linear or branched C₁₋₃-alkyl group; and
R³ and R⁴ represent independently from each other either H or a methyl group.

The term "*Polymer A*" indicates in this document a homopolymer of

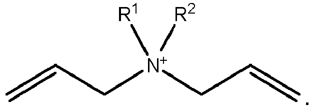

The term *"Polymer B"* indicates in this document a copolymer of

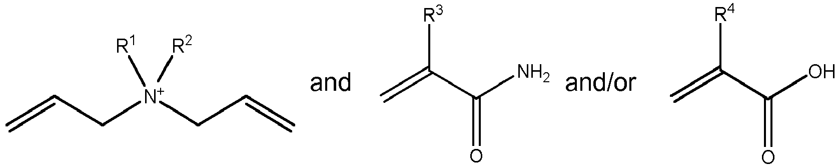

The term "independently from each other" in this document means, in the context of substituents, moieties, or groups, that identically designated substituents, moieties, or groups can occur simultaneously with a different meaning in the same molecule.

A "C_{x-y}-alkyl" group is an alkyl group comprising x to y carbon atoms, i.e., for example, a C₁₋₃-alkyl group is an alkyl group comprising 1 to 3 carbon atoms. The alkyl group can be linear or branched. For example -CH(CH₃)-CH₂-CH₃ is considered as a C₄-alkyl group.

In case identical labels for symbols or groups are present in several formulae, in the present document, the definition of said group or symbol made in the context of one specific formula applies also to other formulae which comprises said same label.

The expression "process of preparation" is a synonym for "method of preparation" and can be used interchangeable to each other.

Ultraviolet light (=UV light) is understood in the present document of electromagnetic radiation of the wavelength between 280 and 400 nm.

"Leave-on treatment", sometimes also called "Leave-in treatment" is a term used and known by the person skilled in the art for any hair treatment in which a cosmetic product is applied to the hair and is not washed out or off the hair thereafter.

It is preferred that R¹ and R² represent identical residues, particularly it is preferred, that R¹ and R² represent each a methyl group.

It is, furthermore, preferred that R³ and R⁴ represent each H.

The homopolymer or copolymer, is obtained from the respective monomers by polymerization. No further polymerizable monomers are present in the polymerization. The process of the polymerization of the monomers and particular the polymerization conditions are known to the person skilled in the art. Particularly the polymerization of the monomers is initiated by free-radical polymerization initiators such as peroxides or azo compounds.

Examples of peroxide are hydrogen peroxide or peroxodisulfates, such as the mono- or di-alkali metal or ammonium salts of peroxide disulfuric acid, for example, its mono- and di-sodium, -potassium or ammonium salts or organic peroxides, such as alkyl hydroperoxides, for example tert-butyl, p-menthyl or cumyl hydroperoxide, tert-butyl perpivalate, and dialkyl or diaryl peroxides, such as di-tert-butyl or di-cumyl peroxide, 2,5-dimethyl-2,5-di(t)butyl-peroxy(hexane) or dibenzoyl peroxide.

Examples of suitable azo compounds are 2,2'-azobis(isobutyronitrile), 2,2'-azobis(2,4-dimethylvaleronitrile) and 2,2'-azobis(amidinopropyl) dihydrochloride, 1,1'-azobis(1-cyclohexanecarbonitrile), 2,2'-azobis(2-amidinopropane) salts, 4,4'-azobis(4-cyanovaleric acid) or 2-(carbamoylazo)isobutyronitrile.

Particularly the polymerization is an emulsion polymerization.

In the case of copolymers, also monomers of different R¹, R², R³ and/or R⁴ can be used. For example, a copolymer of 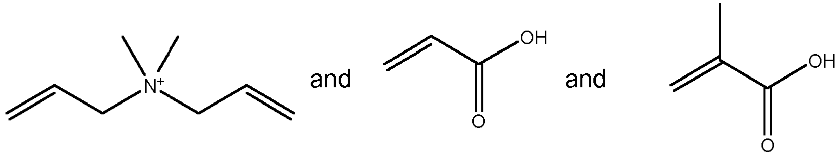 or a copolymer of 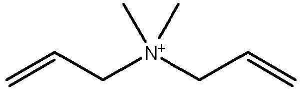 and 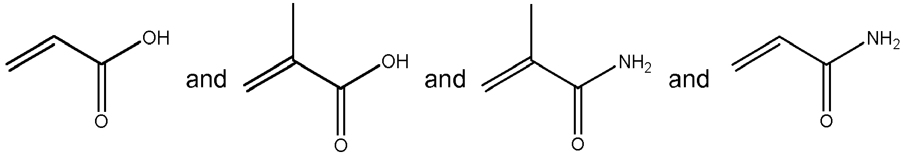 are both regarded as being a copolymer of

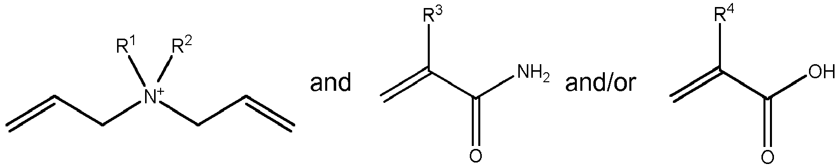

In a preferred embodiment, the cationic polymer having quaternary ammonium groups is selected from the group consisting of polyquaternium-6, polyquaternium-7, polyquaternium-22 and polyquaternium-39.

In an even more preferred embodiment, the cationic polymer having quaternary ammonium groups is polyquaternium-6 or polyquaternium-22.

Polyquaternium-6, polyquaternium-7, polyquaternium-22 and polyquaternium-39 are known substances under the above INCI nomenclature, respectively under their CAS numbers [26062-79-3], [26590-05-6], [53694-17-0] and [25136-75-8] and are commercially available from different suppliers.

These cationic polymers are found to be very beneficial for the UV protection of the hair.

Therefore, in a preferred embodiment the cationic polymer having quaternary ammonium groups is incorporated in a cosmetic composition suitable for topical application on hair, particularly a hair care product, preferably a hair shampoo or a hair conditioner or hair styling product or a hair treatment product.

Hair mask, hair serum, hair balm, hair loss treatments or hair lotions are examples for treatment products.

When incorporated in such a cosmetic composition, it is preferred that the amount of the cationic polymer having quaternary ammonium groups in the cosmetic product is between 0.1 and 15 % by weight, particularly between 0.3 and 10 % by weight, preferably between 0.5 and 7 % by weight, relative to the cosmetic composition.

Beside the cationic polymer as described above the cosmetic composition can have further constituents.

Particularly, the cosmetic composition comprises a cosmetically suitable carrier.

One or more surfactants may be added to the cosmetic composition. When surfactants are present, they are preferably present at a concentration of from 0.1 to 15 weight percent, based on the total weight of the composition. The surfactants which may be used in the cosmetic composition include for example anionic, cationic, nonionic, or amphoteric surfactants. For example, suitable surfactants include sodium laureth sulfate, ammonium laureth sulfate, sodium lauryl sulfate, ammonium lauryl sulfate, cocamidopropyl betaine, disodium cocoamphodiacetate, sodium cocoamphoacetate, disodium laureth sulfosuccinate, sodium cocoyl glutamate, sodium lauroyl methyl isethionate, coco-glucoside, decyl glucoside, coco betaine, PEG-200 hydrogenated glyceryl palmate, PEG-7 glyceryl cocoate, PEG-120 methyl glucose dioloeate, PEG-150 distearate, PEG-80 sorbitan laurate, cocamide MEA, sucrose cocoate isostearamide MIPA, PPG-28 buteth-35, PEG-75 lanolin, perfluoropolymethyl isopropyl ether, octoxynol-9, PEG-25 hydrogenated castor oil, PEG-40 hydrogenated castor oil, polyethylene terephthalate, polyethylene glycol 25 glyceryl trioleate, oleth-3 phosphate, PPG-5-ceteth-10 phosphate, PEG-20 methyl glucose ether, glycereth-7-triacetate, glycereth-7-benzoate, fatty acid ester of polysorbate (TWEEN), or n-alkyl substituted lactam such as n-octyl pyrrolidone, or combinations thereof.

One or more emulsifiers may be added to the cosmetic composition. When emulsifiers are present, they are preferably present at a concentration of from 0.1 to 10.0 weight percent, based on the total weight of the composition. The emulsifiers which may be used in the cosmetic composition include for example anionic, cationic, nonionic, or amphoteric emulsifiers, especially useful are cationic emulsifiers. For example, suitable emulsifiers include behentrimonium chloride, distearyldimonium chloride, cetrimonium chloride, steartrimonium chloride, palmitamidopropyltrimonium chloride, palmitamidopropyl dimethylamine, behentrimonium methosulfate, cocotrimonium methosulfate, brassicyl isoleucinate esylate and brassica alcohol, ceteareth-20, ceteareth-30.

One or more co-emulsifiers may be added to the cosmetic composition. When emulsifiers are present, they are preferably present at a concentration of from 0.1 to 10.0 weight percent, based on the total weight of the composition. The co-emulsifiers which may be used in the cosmetic composition include for example fatty alcohols such as cetyl alcohol, stearyl alcohol, behenyl alcohol, cetearyl alcohol, fatty esters such as cetyl palmitate.

One or more siloxane derivatives may be present in the cosmetic composition. When they are used, they are preferably present in a concentration from 0.001 to 1.0 weight percent, based on the total weight of the composition. The siloxane derivatives include for example dimethicones, phenyl trimethicones, dimethiconols, amodimethicones, alkoxylated dimethicones e.g. PEG-12 dimethicone or methoxy PEG/PPG-7/3 aminopropyl dimethicone.

One or more solvents may be added to the cosmetic composition. The solvents may or may not be VOC (= Volatile Organic Compounds). When solvents are added to the cosmetic composition they preferably comprise 55 weight percent or less, and more preferably 100 weight percent or less, based on the total weight of the composition. Suitable solvents include for example C1 to C12 straight or branched chain alcohols such as methanol, ethanol, isopropanol, or propanol or combinations thereof.

The cosmetic composition typically comprises water.

Preservatives which may be used in the cosmetic composition include for example isothiazolones, benzyl alcohol, chlorphenesin, sodium benzoate, potassium sorbate, phenoxyethanol, parabens such as methylparaben, ethyl-paraben and propylparaben, ethylhexylglycerin, caprylylglycol, methyldibromo glutaronitrile, iodopropinyl butylcarbamate or imidazolidinylurea and diazolidinyl urea. The preservatives are preferably used in an amount of about 0.001 to 2.0 wt.-% based on the total weight of the cosmetic composition.

One or more thickeners may be desirable in the cosmetic composition. Suitable thickeners include for example polycarboxylic acid thickeners such as acrylates/steareth-20 methacrylate copolymer, carbomers, acrylates copolymer, sodium polyacrylates, or acrylates C10-30 acrylate crosspolymer; polyethoxylated urethane thickeners, or polyamide thickeners. Other suitable thickeners are based on natural polymers such as polysaccharides or polyamides and can be chemically modified. Such thickeners include for instance hydroxyethyl celluloses, hydroxypropyl celluloses, microcrystalline cellulose, xanthan gum, gelatine, agar-agar, carragenens, alginates or mixtures thereof. The thickeners are preferably used in an amount of about 0.001 to 5.0 wt.-% based on the total weight of the cosmetic composition.

The cosmetic composition may also comprise further polymers providing beneficial hair attributes such as acrylate copolymers, particularly copolymers of n-butyl methacrylate, methacrylic acids, and ethyl acrylate such as disclosed in detail in WO 2012/072774, particularly as the acrylate copolymer been commercialized as TILAMAR® Fix A140 (CAS:[26715-43-5]) and TILAMAR® Fix A1000 (CAS:[1070166-98-1]) by DSM, Switzerland.

Additionally other additives, such as those commonly used by those skilled in the art may be added to the cosmetic composition. The other additives used in the composition will depend upon the type of cosmetic composition desired. Other additives include for example fragrances; moisturizers such as sorbitol, propane diol, butylene glycol, glycerin, hydrolyzed silk protein, or hydrolyzed wheat protein; detangling aids such as panthenol; conditioning agents such as those disclosed in US Patent 5,164,177, emulsifiers; antistatic aids, extracts, proteins, vitamins, dyes, tints, colorants or combinations thereof.

Further examples of such additives are ingredients to enhance the performance and/or consumer acceptability such as antioxidants, fatty substances/ oils, thickeners, softeners, emulsifiers, antifoaming agents, fillers, sequestering agents, cationic-, nonionic- or amphoteric polymers or mixtures thereof, acidifying or basifying agents, dyes, colorants, pigments or nanopigments, pearlizers or opacifiers such as styrene/acrylates copolymer commercialized as TILAMAR® OP 40 (CAS:[9010-92-8]), organic or inorganic particles, viscosity modifiers, and natural hair nutrients such as botanicals, fruit extracts, sugar derivatives and/or amino acids or any other ingredients usually formulated into hair care compositions. The necessary amounts of the adjuvants and additives can, based on the desired product, easily be chosen by a person skilled in the art in this field and will be illustrated in the examples, without being limited hereto. The other additives are typically present from 0.005 to 10 wt.-%; more preferably from 0.01 to 5 wt.-% based on total weight of the cosmetic composition.

The cosmetic composition may also comprise at least one substance which has beneficial attributes to the scalp or the hair. Such substances are particularly sericin, leontopodium alpinum extract, commercialized under the tradename ALPAFLOR® Edelweiss B, saccharide isomerate, commercialized under the tradename PENTAVITIN®, phytantriol, argania spinose kernel oil and tocopherol and butyrospermum parkii butter extract and spent grain wax, commercialized under the tradename STIMU®-TEX AS, panthenol ethyl ether, commercialized under the tradename Ethyl Panthenol, niacinamide, commercialized under the tradename Niacinamide PC, tocopherol, commercialized under the tradename dl-alpha-Tocopherol, and ceratonia siliqua gum, commercialized under the tradename PHYTALURONATE® PF.

Additional other additives, as well as additional surfactants, solvents, other preservatives, and thickeners, which may be suitable in the cosmetic composition may be found in the International Cosmetic Ingredients Dictionary, 5th Edition, 1993, published by the CTFA in Washington D.C.

It may be advantageous to add also at least one or more UV filters to the above cosmetic composition. The UV filer is advantageously selected from UV-A, UV-B, UV-C and/ or broadband filters such as in particular from the commercially available and widely used UV-filter substances octocrylene (PARSOL® 340), 4-methyl benzylidene camphor (PARSOL® 5000), ethylhexyl methoxycinnamate (PARSOL® MCX), ethylhexyl triazone (Uvinul® T-150), diethylhexyl butamido triazone (Uvasorb® HEB), 2,2'-methylene-bis-(6-(2H-benzotriazole-2-yl)-4-(1,1,3,3,-tetramethylbutyl)-phenol (Tinosorb® M), bis-ethylhexyloxyphenol methoxyphenyl triazine (Tinosorb® S), 2,2-(1,4-phenylene)bis-(1H-benzimidazol-4,6-disulfonic acid (NeoHeliopan® AP), 2-(4-Diethylamino-2-hydroxy-benzoyl)-benzoic acid hexylester (Uvinul® A plus), polysilicone-15 (PARSOL® SLX), 2-phenyl benzimidazole sulfonic acid (PARSOL® HS), ethylhexyl salicylate (PARSOL® EHS), homomenthyl salicylate (PARSOL® HMS), Benzophenone-3 (Uvinul® M 40), Benzophenone-4 (Uvinul® MS 40), PEG-25 PABA, butyl methoxydibenzoyl methane (PARSOL® 1789) as well as mixtures thereof.

Preferred UV Filters are polysilicone-15 (PARSOL® SLX) and ethylhexyl methoxycinnamate (PARSOL® MCX).

The effect of the UV protection can be further improved by adding at least one UV-filter.

Such cosmetic compositions have the same level of UV protection at a significantly lower price due to the fact that less amount of expensive UV filters are needed.

In a further aspect, the present invention relates to a method of protecting hair from UV damage comprising the steps
a) applying a cationic polymer having quaternary ammonium groups as described in great detail above to the surface of hair;
b) exposing the hair to which cationic polymer having quaternary ammonium groups has been applied according to step a) to UV radiation;
c) observing a lower level of hair damages after UV exposure according to step b) as compared to hair which has been exposed to UV radiation according to step b), however, to which no cationic polymer having quaternary ammonium groups has been applied according to step a);

The lower hair damage after UV exposure can be determined by DSC (Differential Scanning Colorimetry) method in having a higher denaturation temperature and higher denaturation enthalpy as compare to the unprotected hair.

It has been observed that already small amounts of cationic polymer having quaternary ammonium groups in a cosmetic composition lead to a significant UV protection of the hair. It has been found that already as little as 1.2 % by weight of cationic polymer having quaternary ammonium groups relative to the weight of the cosmetic composition applied to the hair lead to an increase in denaturation temperature of more than 0.8%, sometimes even more than 5.9 %, as compared to the non-UV-protected hair.

Already as little as between 0.1 and 15 % by weight, particularly between 0.3 and 10 % by weight, preferably between 0.5 and 7 % by weight, relative to the cosmetic composition can achieve significant UV protection of hair.

### Examples

The present invention is further illustrated by the following, non-limiting examples, in which all percentages are by weight based on total weight unless otherwise specified.

### Leave-on alcohol based oily protection pump sprays

In a first series of examples, the compositions according to table 1 have been applied with a syringe (0.8 ml) each onto three tresses of hair (826200, Eurohair -over bleached, 1cm width, free hair length 10 cm (Kerling International) color 10/0, mixture 476) which before have been cleaned by washing them twice using standard cleansing shampoo (HC.E. 100147.001) and towel-dried. After massaging the composition during 2 minutes into the hair tresses and combing the respective tresses have been dried in a climate chamber (20°C, 60 % relative humidity) during 4 hours and exposed to UV irradiation using an Atlas Suntester with 500 W/m² during 150 hours.

After the exposure to UV light, 3 sample of respective hair have been measured by DSC (Differential Scanning Calorimetry)(Discovery DSC, DSC 1-0070 TA instruments) (25mg hair in a high volume pan) between 80°C and 180°C at a heating rate of 10 °C/min. The denaturation temperature T_{D} (DSC peak) is determined using the TA software and listed in table 1. In table 1 also the relative increase of T_{D} relative to the composition having no UV filters and no respective cationic polymer having quaternary ammonium groups (i.e. relative to Ref.1) is given.

It is explicitly stated that the Polyquaternium-6 solution used comprises no stabilizers which might result in a UV protection effect.

**Table 1. The UV protection effect of cationic polymer having quaternary ammonium groups in alcohol/oil based compositions.**

| ***Ingredients (INCI)*** | ***Ref.1*** | ***1*** | ***2*** | ***3*** |
|---|---|---|---|---|
| Polyquaternium-6 (solution; 40% by weight in water) | | 3.0 | 3.0 | 3.0 |
| Polysilicone-15 | | | 3.0 | 3.0 |
| Ethylhexyl Methoxycinnamate | | | | 2.5 |
| water | 45.0 | 45.0 | 45.0 | 45.0 |
| Cetrimonium Chloride | 5.0 | 5.0 | 5.0 | 5.0 |
| Sericin | 2.0 | 2.0 | 2.0 | 2.0 |
| Panthenyl Ethyl Ether | 0.25 | 0.25 | 0.25 | 0.25 |
| Ethanol absolute | 44.35 | 41.35 | 38.35 | 35.85 |
| Undecane (and) Tridecane | 2.2 | 2.2 | 2.2 | 2.2 |
| Argania Spinosa Kernel Oil | 0.5 | 0.5 | 0.5 | 0.5 |
| Parfume | 0.25 | 0.25 | 0.25 | 0.25 |
| PEG-40 Hydrogenated Castor Oil | 0.45 | 0.45 | 0.45 | 0.45 |
| | | | | |
| T_{D} [°C] | 137.6 | 140.2 | 142.7 | 145.9 |
| increase [%] | | +1.9% | +3.7% | *+6.0%* |

The results of table 1 show that the compositions comprising *Polymer A* (***1,2,3***) show an increased denaturation temperature as compared to the respective composition having neither UV filters nor cationic polymer having quaternary ammonium groups (***Ref.1***). Combining *Polymer A* with UV filters (***2,3***) leads to a significant increase in the denaturation temperature.

### Leave-on water based protection pump sprays

In a second series of examples, the compositions according to table 2 have been applied with a syringe (0.8 ml) each onto three tresses of hair (826200, Eurohair -over bleached, 1cm width, free hair length 10 cm (Kerling International) color 10/0, mixture 476) which before have been cleaned by washing them twice using standard cleansing shampoo (HC.E. 100147.001) and towel-dried. After massaging the composition during 2 minutes and combing the respective tresses have been dried in a climate chamber (20°C, 60 % relative humidity) during 4 hours and exposed to UV irradiation using an Atlas Suntester with 500 W/m² during 150 hours.

After the exposure to UV light, 3 sample of respective hair have been measured by DSC (Differential Scanning Calorimetry)(Discovery DSC, DSC 1-0070 TA instruments) (25mg hair in a high volume pan) between 80°C and 180°C at a heating rate of 10°C/min. The denaturation temperature T_{D} (DSC peak) and the denaturation enthalpy ΔH_{D} are determined using the TA software and listed in table 2. In table 2 also the relative increase of T_{D} respectively of ΔH_{D} relative to the composition having no respective cationic polymer having quaternary ammonium groups (i.e. relative to ***Ref.2***) is given.

It is explicitly stated that the Polyquaternium-22 solution used comprises no stabilizers which might result in a UV protection effect.

**Table 2. The UV protection effect of cationic polymer having quaternary ammonium groups in water based compositions.**

| ***Ingredients (INCI)*** | ***Ref.2*** | ***4*** | ***5*** |
|---|---|---|---|
| Polyquaternium-22 (solution 40% by weight in water) | | 3.0 | 3.0 |
| Acrylate Copolymer(solution 40% by weight)¹ | | | 1.25 |
| Water | 93.48 | 90.48 | 89.23 |
| Aminomethyl Propanol | 0.27 | 0.27 | 0.27 |
| Sodium Cocoyl Glutamate | 5.0 | 5.0 | 5.0 |
| Panthenyl Ethyl Ether | 0.25 | 0.25 | 0.25 |
| Ethylhexylglycerin, Phenoxyethanol | 1.0 | 1.0 | 1.0 |
| | | | |
| T_{D} [°C] | 146.3 | 147.6 | 148.9 |
| increase [%] | | *+0.9%* | +1.8% |
| ΔH_{D} [J/g] | 9.0 | 15 | 15.8 |
| increase [%] | | +66.7% | +75.6% |

| | | | |
|---|---|---|---|
| ¹ TILAMAR® Fix A140 | | | |

The results of table 2 show that the compositions comprising *Polymer B* (***4,5***) show an increased denaturation temperature and denaturation enthalpy as compared to the respective composition having no respective cationic polymer having quaternary ammonium groups (***Ref.2***)

### Leave-on water based protection pump sprays

In a third series of examples, no product (***Ref.3***) or the compositions according to table 3 (***6,7***) have been applied with a syringe (0.8 ml) each onto three tresses of hair (826200, Eurohair -over bleached, 1cm width, free hair length 10 cm (Kerling International) color 10/0, mixture 476) which before have been cleaned by washing them twice using standard cleansing shampoo (HC.E. 100147.001) and towel-dried. After massaging the composition during 2 minutes and combing the respective tresses have been dried in a climate chamber (20°C, 60 % relative humidity) during 4 hours and exposed to UV irradiation using an Atlas Suntester with 500 W/m² during 150 hours.

After the exposure to UV light, 3 sample of respective hair have been measured by DSC (Differential Scanning Calorimetry)(Discovery DSC, DSC 1-0070 TA instruments) (25mg hair in a high volume pan) between 80°C and 180°C at a heating rate of 10°C/min. The denaturation temperature T_{D} (DSC peak) and the denaturation enthalpy ΔH_{D} are determined using the TA software and listed in table 3. In table 3 also the relative increase of T_{D} respectively of ΔH_{D} relative to the hair not been treated with a composition having respective cationic polymer having quaternary ammonium groups (i.e. relative to ***Ref.3***) is given.

It is explicitly stated that the Polyquaternium-6 solution and Polyquaternium-22 solution used comprise no stabilizers which might result in a UV protection effect.

**Table 3. The UV protection effect of cationic polymer having quaternary ammonium groups in water based compositions.**

| ***Ingredients (INCI)*** | ***Ref.3*** | ***6*** | ***7*** |
|---|---|---|---|
| Polyquaternium-6 (solution 40% by weight in water) | | 3.0 | |
| Polyquaternium-22 (solution 40% by weight in water) | | | 3.0 |
| Ethylhexylglycerin, Phenoxyethanol | | 0.5 | 0.5 |
| Water | | 96.5 | 96.5 |
| | | | |
| T_{D} [°C] | 141.1 | 151.3 | 148.9 |
| increase [%] | | +7.2% | +5.5% |
| | | | |
| ΔH_{D} [J/g] | 7.5 | 8.8 | n.d.¹ |
| increase [%] | | *+17.3%* | *n.d.*¹ |

| | | | |
|---|---|---|---|
| ¹ n.d.= not determined | | | |

The results of table 3 show that the compositions comprising *Polymer A* or *Polymer B* (***6,7***) show a significantly increased denaturation temperature and denaturation enthalpy ΔH_{D} as compared to the UV damaged hair not been treated with compositions having the respective polymers having quaternary ammonium groups (***Ref.3***).

## Claims

1. A use of a cationic polymer having quaternary ammonium groups which is either a homopolymer of 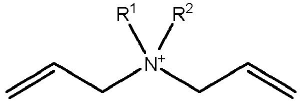 or a copolymer of 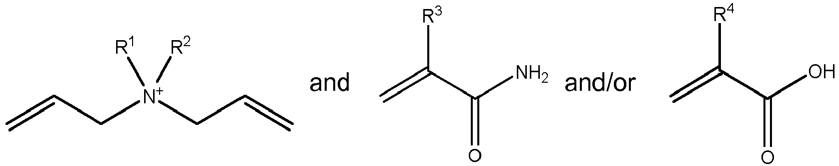 for the protection of hair against UV damage;
wherein
R¹ and R² represent independently from each other either a linear or a branched C₁₋₁₀-alkyl group, particularly a linear or branched C₁₋₃-alkyl group; and
R³ and R⁴ represent independently from each other either H or a methyl group.

2. The use according to claim 1, **characterized in that** R¹ and R² represent each a methyl group.

3. The use according to claim 1 or 2, **characterized in that** R³ and R⁴ represent each H.

4. The use according to anyone of the preceding claims, **characterized in that** the cationic polymer having quaternary ammonium groups is selected from the group consisting of polyquaternium-6, polyquaternium-7, polyquaternium-22 and polyquaternium-39.

5. The use according to anyone of the preceding claims, **characterized in that** the cationic polymer having quaternary ammonium groups is polyquaternium-6 or polyquaternium-22.

6. The use according to anyone of the preceding claims, **characterized in that** the cationic polymer having quaternary ammonium groups is incorporated in a cosmetic composition suitable for topical application on hair, particularly a hair care product, preferably a hair shampoo or a hair conditioner or hair styling product or a hair treatment product.

7. The use according to claim 6, **characterized in that** the amount of the cationic polymer having quaternary ammonium groups in the cosmetic product is between 0.1 and 15 % by weight, particularly between 0.3 and 10 % by weight, preferably between 0.5 and 7 % by weight, relative to the cosmetic composition.

8. A method of protecting hair from UV damage comprising the steps:
a) applying a cationic polymer having quaternary ammonium groups to the surface of hair;
b) exposing the hair to which cationic polymer having quaternary ammonium groups has been applied according to step a) to UV radiation;
c) observing a lower level of hair damages after UV exposure according to step b) as compared to hair which has been exposed to UV radiation according to step b), however, to which no cationic polymer having quaternary ammonium groups has been applied according to step a);
**characterized in that** the cationic polymer having quaternary ammonium groups is
either a homopolymer of 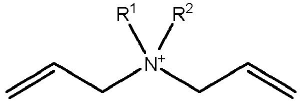 or a copolymer of 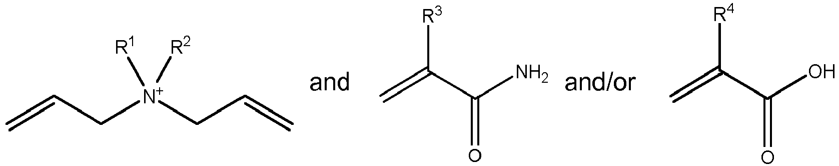 wherein
R¹ and R² represent independently from each other either a linear or a branched C₁₋₁₀-alkyl group, particularly a linear or branched C₁₋₃-alkyl group; and
R³ and R⁴ represent independently from each other either H or a methyl group.

9. The method according to claim 8, **characterized in that** R¹ and R² represent each a methyl group.

10. The method according to claim 8 or 9, **characterized in that** R³ and R⁴ represent each H.

## Patentansprüche

1. Verwendung eines kationischen Polymers mit quartären Ammoniumgruppen, bei dem es sich entweder um ein Homopolymer von 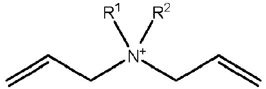 oder um ein Copolymer von 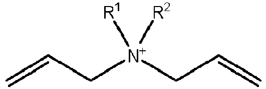 und 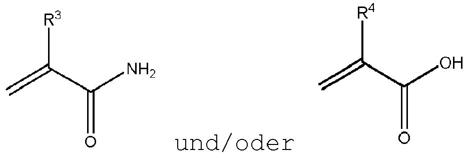 handelt, zum Schutz von Haar gegen UV-Schädigung;
wobei
R¹ und R² unabhängig voneinander für eine lineare oder verzweigte C₁₋₁₀-Alkylgruppe, insbesondere eine lineare oder verzweigte C₁₋₃-Alkylgruppe, stehen und
R³ und R⁴ unabhängig voneinander entweder für H oder für eine Methylgruppe stehen.

2. Verwendung nach Anspruch 1, **dadurch gekennzeichnet, dass** R¹ und R² jeweils für eine Methylgruppe stehen.

3. Verwendung nach Anspruch 1 oder 2, **dadurch gekennzeichnet, dass** R³ und R⁴ jeweils für H stehen.

4. Verwendung nach einem der vorhergehenden Ansprüche, **dadurch gekennzeichnet, dass** das kationische Polymer mit quartären Ammoniumgruppen aus der Gruppe bestehend aus Polyquaternium-6, Polyquaternium-7, Polyquaternium-22 und Polyquaternium-39 ausgewählt ist.

5. Verwendung nach einem der vorhergehenden Ansprüche, **dadurch gekennzeichnet, dass** es sich bei dem kationischen Polymer mit quartären Ammoniumgruppen um Polyquaternium-6 oder Polyquaternium-22 handelt.

6. Verwendung nach einem der vorhergehenden Ansprüche, **dadurch gekennzeichnet, dass** das kationische Polymer mit quartären Ammoniumgruppen in eine kosmetische Zusammensetzung eingearbeitet ist, die für die topische Anwendung auf Haar geeignet ist, insbesondere ein Haarpflegeprodukt, vorzugsweise ein Haarshampoo oder ein Haarconditioner oder ein Haarstylingsprodukt oder ein Haarbehandlungsprodukt.

7. Verwendung nach Anspruch 6, **dadurch gekennzeichnet, dass** die Menge des anionischen Polymers mit quartären Ammoniumgruppen in dem kosmetischen Produkt zwischen 0,1 und 15 Gew.-%, insbesondere zwischen 0,3 und 10 Gew.-%, vorzugsweise zwischen 0,5 und 7 Gew.-%, bezogen auf die kosmetische Zusammensetzung, liegt.

8. Verfahren zum Schützen von Haar vor UV-Schädigung, bei dem man:
a) ein kationisches Polymer mit quartären Ammoniumgruppen auf die Oberfläche des Haars aufbringt;
b) das Haar, auf das kationisches Polymer mit quartären Ammoniumgruppen gemäß Schritt a) aufgebracht wurde, UV-Strahlung aussetzt;
c) ein geringeres Niveau von Haarschädigungen nach UV-Exposition gemäß Schritt b) im Vergleich zu Haar, das UV-Strahlung gemäß Schritt b) ausgesetzt wurde, auf das aber kein kationisches Polymer mit quartären Ammoniumgruppen gemäß Schritt a) aufgebracht wurde;
**dadurch gekennzeichnet, dass** es sich bei dem kationischen Polymer mit quartären Ammoniumgruppen entweder um ein Homopolymer von 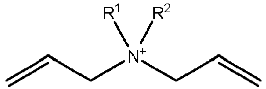 oder um ein Copolymer von 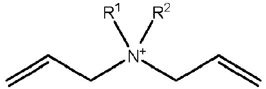 und 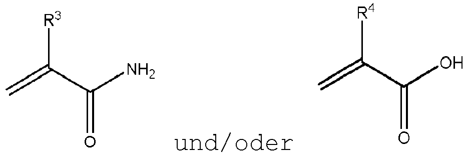 handelt, wobei
R¹ und R² unabhängig voneinander für eine lineare oder verzweigte C₁₋₁₀-Alkylgruppe, insbesondere eine lineare oder verzweigte C₁₋₃-Alkylgruppe, stehen und
R³ und R⁴ unabhängig voneinander entweder für H oder für eine Methylgruppe stehen.

9. Verfahren nach Anspruch 8, **dadurch gekennzeichnet, dass** R¹ und R² jeweils für eine Methylgruppe stehen.

10. Verfahren nach Anspruch 8 oder 9, **dadurch gekennzeichnet, dass** R³ und R⁴ jeweils für H stehen.

## Revendications

1. Utilisation d'un polymère cationique ayant des groupes ammonium quaternaire qui est
soit un homopolymère de 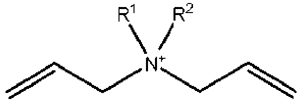 ou un copolymère de 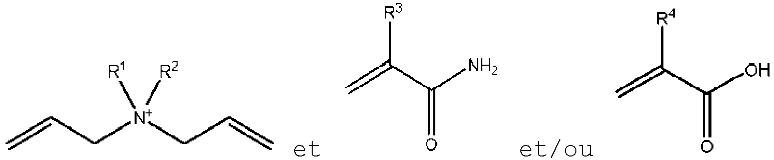 pour la protection des cheveux contre les dommages dus aux UV ;
dans lequel
R¹ et R² représentent indépendamment l'un de l'autre un groupe alkyle en C₁₋₁₀ linéaire ou ramifié, en particulier un groupe alkyle en C₁₋₃ linéaire ou ramifié ; et
R³ et R⁴ représentent indépendamment l'un de l'autre H ou un groupe méthyle.

2. Utilisation selon la revendication 1, **caractérisée en ce que** R¹ et R² représentent chacun un groupe méthyle.

3. Utilisation selon la revendication 1 ou 2, **caractérisée en ce que** R³ et R⁴ représentent chacun H.

4. Utilisation selon l'une quelconque des revendications précédentes, **caractérisée en ce que** le polymère cationique ayant des groupes ammonium quaternaire est choisi dans le groupe constitué de polyquaternium-6, polyquaternium-7, polyquaternium-22 et polyquaternium-39.

5. Utilisation selon l'une quelconque des revendications précédentes, **caractérisée en ce que** le polymère cationique ayant des groupes ammonium quaternaire est polyquaternium-6 ou polyquaternium-22.

6. Utilisation selon l'une quelconque des revendications précédentes, **caractérisée en ce que** le polymère cationique ayant des groupes ammonium quaternaire est incorporé dans une composition cosmétique adaptée pour application topique sur les cheveux, en particulier un produit de soin capillaire, de préférence un shampoing capillaire ou un conditionneur capillaire ou un produit de coiffure des cheveux ou un produit de traitement capillaire.

7. Utilisation selon la revendication 6, **caractérisée en ce que** la quantité du polymère cationique ayant des groupes ammonium quaternaire dans le produit cosmétique est comprise entre 0,1 et 15 % en poids, en particulier entre 0,3 et 10 % en poids, de préférence entre 0,5 et 7 % en poids, par rapport à la composition cosmétique.

8. Procédé de protection des cheveux contre les dommages dus aux UV comprenant les étapes de :
a) application d'un polymère cationique ayant des groupes ammonium quaternaire sur la surface des cheveux ;
b) exposition des cheveux sur lesquels un polymère cationique ayant des groupes ammonium quaternaire a été appliqué conformément à l'étape a) à un rayonnement UV ;
c) l'observation d'un taux plus faible de dommages sur les cheveux après exposition aux UV conformément à l'étape b) par rapport à des cheveux qui ont été exposés à un rayonnement UV conformément à l'étape b), mais sur lesquels aucun polymère cationique ayant des groupes ammonium quaternaire n'a été appliqué conformément à l'étape a) ;
**caractérisé en ce que** le polymère cationique ayant des groupes ammonium quaternaire est
soit un homopolymère de 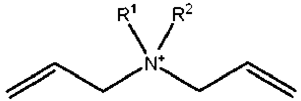 ou un copolymère de 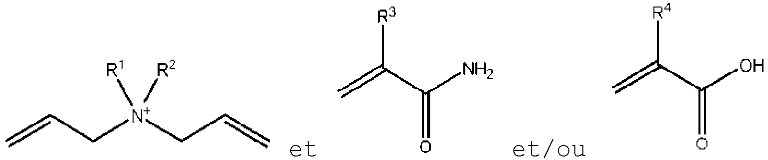 dans lequel
R¹ et R² représentent indépendamment l'un de l'autre un groupe alkyle en C₁₋₁₀ linéaire ou ramifié, en particulier un groupe alkyle en C₁₋₃ linéaire ou ramifié ; et
R³ et R⁴ représentent indépendamment l'un de l'autre H ou un groupe méthyle.

9. Procédé selon la revendication 8, **caractérisé en ce que** R¹ et R² représentent chacun un groupe méthyle.

10. Procédé selon la revendication 8 ou 9, **caractérisé en ce que** R³ et R⁴ représentent chacun H.
